# EUROPÄISCHE PATENTSCHRIFT

(11) **EP 2 088 943 B1**
(45) Veröffentlichungstag und Bekanntmachung des Hinweises auf die Patenterteilung: **11.08.2010**
(21) Anmeldenummer: 07819347.1
(22) Anmeldetag: 26.10.2007
(51) Int. Cl.: A61B 17/34, A61B 19/00

(54) **CHIRURGISCHER OBTURATOR**
SURGICAL OBTURATOR
OBTURATEUR CHIRURGICAL

(30) Priorität: 14.12.2006 DE 102006059012
(43) Veröffentlichungstag der Anmeldung: 19.08.2009
(60) Teilanmeldung: 09161399.2
(73) Patentinhaber: Aesculap AG, 78532 Tuttlingen (DE)
(72) Erfinder: MAYENBERGER, Rupert, 78239 Rielasingen (DE); MALIGLOWKA, Johann, 78600 Kolbingen (DE)
(74) Vertreter: Hoeger, Stellrecht & Partner Patentanwälte
(86) Internationale Anmeldenummer: PCT/EP2007/009299
(87) Internationale Veröffentlichungsnummer: WO 2008/071257

(56) Entgegenhaltungen:
- EP-A- 0 495 633
- DE-U1-202006 008 405
- DE-U1-202006 018 883
- US-A- 5 346 459
- US-A- 5 360 405

## Beschreibung

Die Erfindung betrifft einen chirurgischen Obturator zum Durchstechen einer Körperwand mit einem rohrförmigen Gehäuse, mit einem in dem rohrförmigen Gehäuse in Längsrichtung zwischen einer vorgeschobenen Schneidstellung und einer zurückgezogenen Schutzstellung verschieblichen Messerträger, an dem ein Messer mit einer Schneidkante gehalten ist, die in einer entsprechend verlaufenden Führung des rohrförmigen Gehäuses geführt ist, mit einer das Messer überfangenden, im rohrförmigen Gehäuse in Längsrichtung zwischen einer vorgeschobenen Ruhestellung, in der das Messer in seiner Schutzstellung vollständig überdeckt ist, und einer zurückgezogenen Arbeitsstellung verschiebbaren Schutzkappe, die einen Schlitz aufweist, durch welchen die Schneidkante des Messers hindurchtritt und in der Arbeitsstellung der Schutzkappe über diese hervorsteht, und mit einer in dem rohrförmigen Gehäuse angeordneten, das Messer aus der Schneidstellung in die Schutzstellung verschiebenden Rückzugseinrichtung, die durch Verschiebung der Schutzkappe von der Arbeitsstellung in die Ruhestellung aktivierbar ist.

Ein solcher chirurgischer Obturator ist im deutschen Gebrauchsmuster 20 2006 008 405 U1 beschrieben. Die Rückzugseinrichtung ist dabei im Griffbereich des Obturators angeordnet, in dem relativ viel Platz für eine aufwendige Mechanik der Rückzugseinrichtung zur Verfügung steht.

Auch bei anderen bekannten chirurgischen Obturatoren sind derartige Rückzugseinrichtungen bekannt, die dafür sorgen, dass beim vollständigen Durchtrennen der Körperwand das Messer oder eine Trokarspitze automatisch zurückgezogen werden. In allen Fällen sind die Aufbauten der Rückzugseinrichtungen kompliziert, diese Rückzugseinrichtungen weisen viele Teile auf und haben einen großen Platzbedarf, so dass sie in allen Fällen in einem von dem Messer oder der Trokarspitze entfernten Bereich angeordnet werden müssen, teilweise im Griff des Obturators (EP 0 499 457 B1; EP 0 705 077 B1; EP 0 600 921 B1) oder in einer sperrigen Spezialkonstruktion (US 5,462,532 A).

In der US 5,360,405 ist ein chirurgischer Obturator beschrieben, bei dem die Rückzugseinrichtung ein im rohrförmigen Gehäuse gelagertes, um die Längsachse des rohrförmigen Gehäuses verdrehbares Schaltelement aufweist, welches das Messer aus der Schneidstellung in die Schutzstellung verschiebt und welches bei einer Verschiebung des Messerträgers in Längsrichtung des rohrförmigen Gehäuses über eine zwischen dem Schaltelement einerseits und dem Messerträger andererseits wirksame Nockenführung verdrehbar ist. Auch bei dieser Anordnung ergibt sich ein relativ großer Platzbedarf für die Rückzugseinrichtung.

Es ist Aufgabe der Erfindung, einen gattungsgemäßen chirurgischen Obturator so auszubilden, dass die Rückzugseinrichtung weniger kompliziert aufgebaut ist und einen geringeren Platzbedarf als bekannte Rückzugseinrichtungen erfordert.

Diese Aufgabe wird bei einem chirurgischen Obturator der eingangs beschriebenen Art erfindungsgemäß dadurch gelöst, dass die Rückzugseinrichtung ein das Messer in der Schneidstellung fixierendes und bei Aktivierung für eine Verschiebung in die Ruhestellung freigebendes, um die Längsachse des rohrförmigen Gehäuses frei drehbar und in Längsrichtung unverschieblich im rohrförmigen Gehäuse gelagertes Schaltelement aufweist, welches bei einer Verschiebung des Messerträgers und/oder der Schutzkappe in Längsrichtung des rohrförmigen Gehäuses über eine zwischen dem Schaltelement einerseits und dem Messerträger und/oder der Schutzkappe andererseits wirksame Nockenführung verdrehbar ist, und dass die Schutzkappe und der Messerträger von einer diese auseinander schiebenden gemeinsamen Feder beaufschlagt sind, welche die Schutzkappe von der Arbeitsstellung in die Ruhestellung und den Messerträger von der Schneidstellung in die Schutzstellung verschiebt.

Es werden also die Bewegungen der Schutzkappe und des Messerträgers, die im Wesentlichen in Längsrichtung des rohrförmigen Gehäuses erfolgen, verwendet, um ein Schaltelement über Nockenführungen um die Längsachse des rohrförmigen Gehäuses zu verdrehen. Diese Drehbewegung des Schaltelementes wird ausgenützt, um den Messerträger in der Schneidstellung zu fixieren und wieder freizugeben, sobald die Schutzkappe nach dem Durchdringen der Körperwand von der Arbeitsstellung in die Ruhestellung verschoben wird. Die Umsetzung der Verschiebung des Messerträgers und der Schutzkappe in eine Drehbewegung des Schaltelementes macht es möglich, einen kompakten Aufbau mit nur wenigen Einzelteilen zu erzielen, so dass dieser Rückzugsmechanismus in dem rohrförmigen Gehäuse selbst untergebracht werden kann, vorzugsweise unmittelbar im Bereich des Messerträgers. Dazu ist es nicht notwendig, die Außenabmessungen des rohrförmigen Gehäuses zu erhöhen oder spezielle Aufnahmeräume für die Rückzugseinrichtung vorzusehen.

Bei dieser Konstruktion werden die Schutzkappe und der Messerträger von einer diese auseinander schiebenden gemeinsamen Feder beaufschlagt, welche einerseits die Schutzkappe von der Arbeitsstellung in die Ruhestellung und andererseits den Messerträger von der Schneidstellung in die Schutzstellung verschiebt. Dieser Feder kommt bei dieser Konstruktion eine Doppelfunktion zu.

Die Verschiebung der Schutzkappe in die Arbeitsstellung erfolgt gegen die Kraft dieser Feder. Diese Feder ist auch verantwortlich dafür, dass die Schutzkappe nach dem vollständigen Durchdringen der Körperwand von der Körperwand nicht mehr zurückgehalten wird, sondern durch die von dem Obturator erzeugte Öffnung in die Ruhestellung vorgeschoben wird. Die Feder sorgt auch dafür, dass der Messerträger nach Abschluss des Schneidvorganges und durch die Verschiebung der Schutzkappe von der Arbeitsstellung in die Ruhestellung, die die Rückzugseinrichtung aktiviert, von der Schneidstellung in die Schutzstellung zurückgeschoben wird.

Es ist dabei besonders vorteilhaft, wenn die Feder eine im Inneren des Messerträgers angeordnete Schraubenfeder ist, die sich an einem durch die Wand des Messerträgers in dessen Innenraum eintretenden Vorsprung der Schutzkappe abstützt. Man erhält auf diese Weise eine sehr kompakte Anordnung von Schutzkappe, Messerträger und Feder.

Die Schneidkante des Messers kann geradlinig verlaufen, gemäß einer bevorzugten Ausführungsform ist jedoch vorgesehen, dass die Schneidkante des Messers schraubenlinienförmig ist, so dass der Messerträger bei Verschiebung in Längsrichtung relativ zum rohrförmigen Gehäuse und die Schutzkappe bei einer Längsverschiebung relativ zum Messerträger relativ zueinander um die Längsachse des Messerträgers verdreht werden. Diese Verdrehung erfolgt durch die Führung der Schneidkante in dem rohrförmigen Gehäuse und durch die Führung der Schutzkappe, die diese durch die durch den Schlitz in der Schutzkappe hindurchtretende Schneidkante erfährt. Eine schraubenförmige Führung der Schneidkante hat den Vorteil, dass der Operateur beim Durchstechen der Körperwand dies mit einer kombinierten Vorschub- und Drehbewegung besonders gefühlvoll durchführen kann, außerdem hat diese Ausgestaltung den Vorteil, dass auch der Anteil der Drehbewegung, den der Messerträger und die Schutzkappe erfahren, zur Verdrehung des Schaltelementes beitragen können.

Der Messerträger kann vorteilhafterweise einen hülsenförmigen Schaft aufweisen, der konzentrisch zum rohrförmigen Gehäuse angeordnet ist.

Günstig ist es, wenn der Messerträger mit einer Schubstange verbunden ist, die durch das rohrförmige Gehäuse bis zu einer Druckeinrichtung an dem dem Messer gegenüberliegenden Ende des rohrförmigen Gehäuses verläuft. Durch diese Schubstange, die beispielsweise mit einem Druckknopf versehen sein kann, kann der Messerträger im rohrförmigen Gehäuse in Längsrichtung nach vorne verschoben werden, so dass dadurch der Messerträger von der zurückgezogenen Schutzstellung in die vorgeschobene Schneidstellung gelangt.

Gemäß einer besonders bevorzugten-Ausführungsform ist vorgesehen, dass das Schaltelement eine den Messerträger konzentrisch umgebende Hülse ist. Diese konzentrische Anordnung erlaubt die Unterbringung der Teile auf kleinstem Raum und ermöglicht außerdem eine optimale Funktion der zwischen Messerträger und Schaltelement wirksamen Nockenführung.

Es kann nämlich vorgesehen sein, dass die zwischen dem Schaltelement und dem Messerträger wirksame Nockenführung durch Nockenbahnen und daran anlegbare Nocken gebildet werden, die an der Außenseite des Messerträgers bzw. der Innenwand des hülsenförmigen Schaltelementes angeordnet sind.

Insbesondere ist es vorteilhaft, wenn längs des Umfanges des Messerträgers und des hülsenförmigen Schaltelementes mehrere gleichartige Nockenbahnen und Nocken angeordnet sind derart, dass die Nocken bei aufeinanderfolgenden Arbeitszyklen jeweils an einer benachbarten Nockenbahn anliegen. Das Schaltelement wird also bei einem Arbeitszyklus um einen bestimmten Winkelbetrag weitergeschaltet, und nach einem solchen Arbeitszyklus liegen die Nocken dann an der nachfolgenden, gleich aufgebauten Nockenbahn an. Ein Arbeitszyklus ist dabei **dadurch gekennzeichnet, dass** der Messerträger einmal aus seiner zurückgezogenen Schutzstellung in die vorgeschobene Schneidstellung verschoben, dort blockiert und nach dem vollständigen Durchstechen der Körperwand durch Verschiebung der Schutzkappe von der Arbeitsstellung in die Ruhestellung wieder freigegeben und in seine zurückgezogene Schutzstellung zurückgeschoben wird, so dass die Anordnung wieder den Anfangszustand erreicht.

Bei einer besonders bevorzugten Ausführungsform ist vorgesehen, dass jeder Nockenbahn zwei Nocken zugeordnet sind, die beim Verschieben des Messerträgers relativ zu dem Schaltelement nacheinander an der Nockenbahn anliegen. Diese Nocken übernehmen bei der Verschiebebewegung des Messerträgers nacheinander eine Verdrehung des Schaltelementes.

Die Nockenbahn kann durch den Rand eines inselförmigen Vorsprunges gebildet werden, an dem die Nocken bei einem Arbeitszyklus entlanggleiten und dadurch eine Drehbewegung des Schaltelementes erzeugen.

Bei einer besonders bevorzugten Ausführungsform ist vorgesehen, dass ein dem Messer zugewandter vorderer Nocken beim Verschieben des Messerträgers aus der Schutzstellung in Richtung auf die Schneidstellung an einem schräg zur Verschieberichtung des Messerträgers verlaufenden ersten Abschnitt der Nockenbahn entlanggleitet und dadurch das Schaltelement ausgehend von einer Ausgangsposition in einer ersten Richtung verdreht, dass ein dem Messer abgewandter hinterer Nocken an einem zweiten in der entgegengesetzten Richtung schräg zur Verschieberichtung des Messerträgers verlaufenden Abschnitt der Nockenbahn zur Anlage gelangt, sobald der vordere Nocken an dem ersten Abschnitt der Nockenbahn vorbeigeschoben ist, so dass das Schaltelement in der entgegengesetzten Richtung in eine Blockierposition verdreht wird, in der beim anschließenden Zurückschieben des Messerträgers aus einer Endstellung, die vor der Schneidstellung liegt, ein an diesem angeordneter Anschlag an einem Anschlag am Schaltelement anstößt und dadurch ein weiteres Zurückschieben des Messerträgers verhindert. Bei dieser Anordnung wird also der Messerträger nicht nur aus der zurückgezogenen Schutzstellung bis in die vorgeschobenen Schneidstellung vorgeschoben, sondern noch darüber hinaus. Diese weitere Verschiebung über die Schneidstellung hinaus dient dazu, das Schaltelement in eine Blockierposition zu verdrehen, so dass beim Zurückziehen des Messerträgers in Richtung auf die Schneidstellung eine weitere Rückbewegung des Messerträgers durch die Anschläge blockiert wird, d.h. das Messer bleibt in einer vorgeschobenen Stellung stehen.

Günstig ist es, wenn der Anschlag am Messerträger durch den vorderen Nocken gebildet wird. Es wird dann kein separater Anschlag benötigt, so dass der Aufbau weiter vereinfacht wird.

Der Anschlag am Schaltelement kann durch einen quer zur Längsrichtung des rohrförmigen Gehäuses verlaufenden dritten Abschnitt der Nockenbahn gebildet werden. Auch dies trägt zur Vereinfachung des Aufbaues bei.

Es kann weiterhin vorgesehen sein, dass die Nockenbahn und die Nocken zwischen dem Schaltelement und der Schutzkappe durch die seitlichen Flanken von Zähnen gebildet werden, die an den einander zugewandten Stirnseiten der Schutzkappe und des Schaltelementes angeordnet sind und die bei in die Arbeitsstellung zurückgeschobener Schutzkappe im Eingriff stehen. Sowohl die Schutzkappe als auch das Schaltelement tragen also an ihren einander zugewandten Stirnseiten Zähne, die nicht in Eingriff stehen, solange die Schutzkappe in ihrer vorgeschobenen Ruhestellung steht, die aber in Eingriff gelangen, sobald die Schutzkappe in die Arbeitsstellung zurückgezogen wird.

Die Flanken der Zähne können so angeordnet und ausgebildet sind, dass die Zähne der Schutzkappe beim Zurückziehen der Schutzkappe von der Ruhestellung in die Arbeitsstellung das Schaltelement aus seiner Blockierposition in der ersten Richtung verdrehen, bis der Anschlag des Messerträgers auf einen schräg zur Längsrichtung des rohrförmigen Gehäuses verlaufenden vierten Abschnitt der Nockenbahn des Schaltelementes gelangt, der sich an den Anschlag des Schaltelements anschließt. Sobald der Anschlag des Messerträgers an diesem vierten Abschnitt anliegt, übt der Messerträger unter der Wirkung der auf ihn wirkenden Feder eine Kraft auf das Schaltelement aus und versucht dieses in der zweiten Richtung zu verdrehen.

Eine solche Verdrehung wird jedoch gemäß einer weiteren bevorzugten Ausführungsform dadurch verhindert, dass die Flanken der Zähne so ausgebildet sind, dass eine Drehung des Schaltelementes in der zweiten Richtung unter dem Einfluss des am vierten Abschnitt anliegenden Anschlages des Messerträgers derart begrenzt wird, dass der Messerträger gegenüber seiner Stellung bei Anlage der beiden Anschläge aneinander nur wenig zurückgeschoben wird bis in seine Schneidstellung. Der Messerträger wird also in einer Schneidstellung blockiert, die nur ganz wenig gegenüber der Blockierstellung zurückgeschoben ist, in dieser Schneidstellung kann das Durchstechen der Körperwand erfolgen, da der Messerträger durch das Schaltelement am Zurückschieben in Richtung auf die Schutzstellung gehindert ist.

Es kann weiterhin vorgesehen sein, dass der Eingriff der Zähne des Messerträgers und der Schutzkappe bei Verschiebung der Schutzkappe in die Ruhestellung aufgehoben wird, so dass die Begrenzung der Verdrehbarkeit des Schaltelementes aufgehoben wird. Sobald dies eintritt, kann der Messerträger unter dem Einfluss der Feder in die Schutzstellung zurückgeschoben werden, d.h. die Verschiebung der Schutzkappe in die Ruhestellung löst die Rückbewegung des Messerträgers aus.

Es ist dabei vorteilhaft, wenn die Nockenbahn einen sich an den schrägen vierten Abschnitt anschließenden fünften Abschnitt aufweist, an dem der vordere Nocken des Messerträgers beim Zurückschieben des Messerträgers aus der Schneidstellung in die Schutzstellung entlanggleitet und das Schaltelement dabei in der zweiten Richtung in die Ausgangsposition für den nächsten Arbeitszyklus verdreht.

Gemäß einer weiteren bevorzugten Ausführungsform steht der hintere Nocken des Messerträgers bei der Stellung des Schaltelementes in der Blockierposition und bei Anlage des Anschlages des Messerträgers an dem vierten Abschnitt der Nockenbahn einem sechsten Abschnitt der Nockenbahn gegenüber, der eine Verschiebung des Messerträgers in Richtung von der Schutzstellung zur Schneidstellung verhindert. Damit ist das Messer in seiner Lage in der Schneidstellung in beiden Richtungen fixiert, es kann also auch nicht unbeabsichtigt durch Betätigung der Schubstange das Messer über die Schneidstellung hinweg nach vorne verschoben werden.

Die nachfolgende Beschreibung bevorzugter Ausführungsformen der Erfindung dient im Zusammenhang mit der Zeichnung der näheren Erläuterung. Es zeigen:

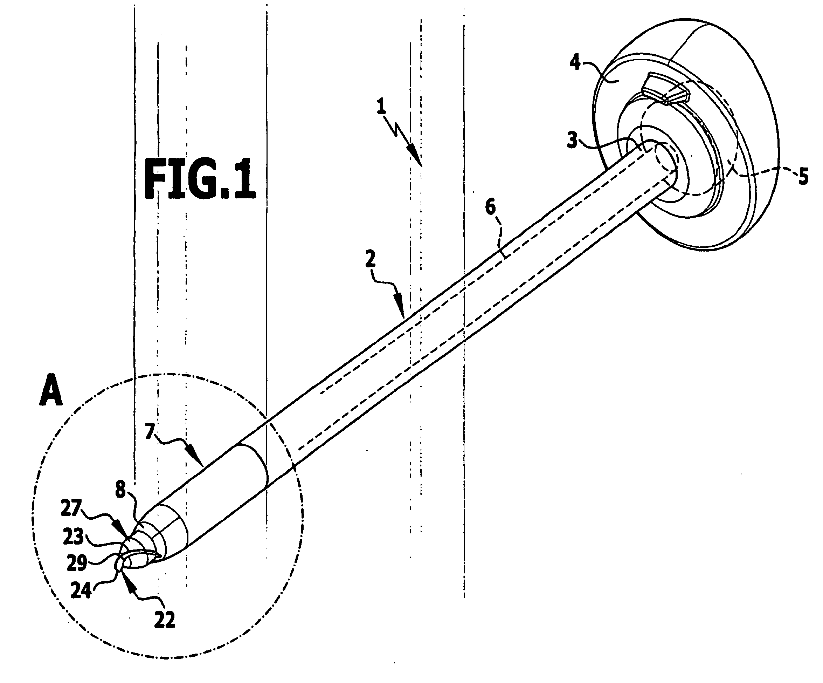

- Figur 1:: eine perspektivische Ansicht eines chirurgischen Obturators mit einem rohrförmigen Gehäuse und einem Messer mit schrauben- linienförmiger Schneidkante;

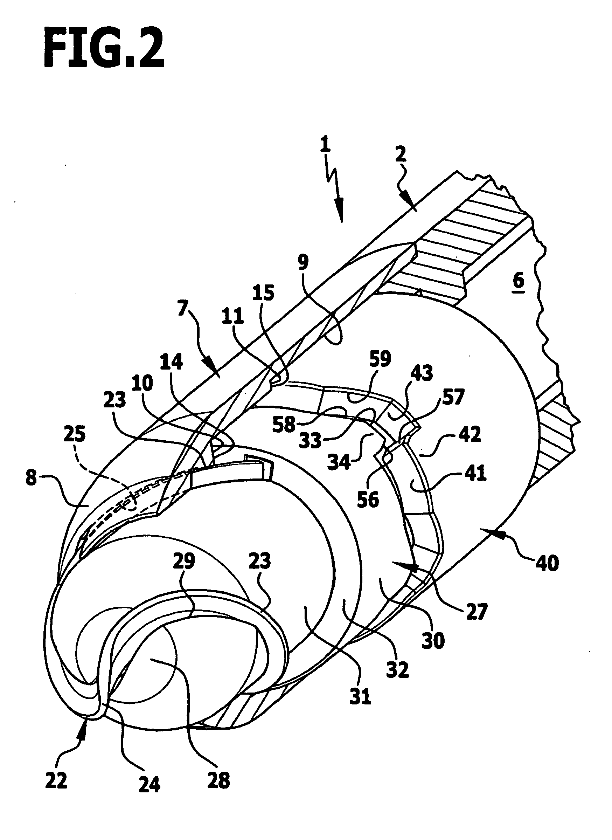

- Figur 2:: eine vergrößerte Detailansicht des Bereiches A in Figur 1 mit in Längsrichtung geschnittenem rohrförmigem Gehäuse;

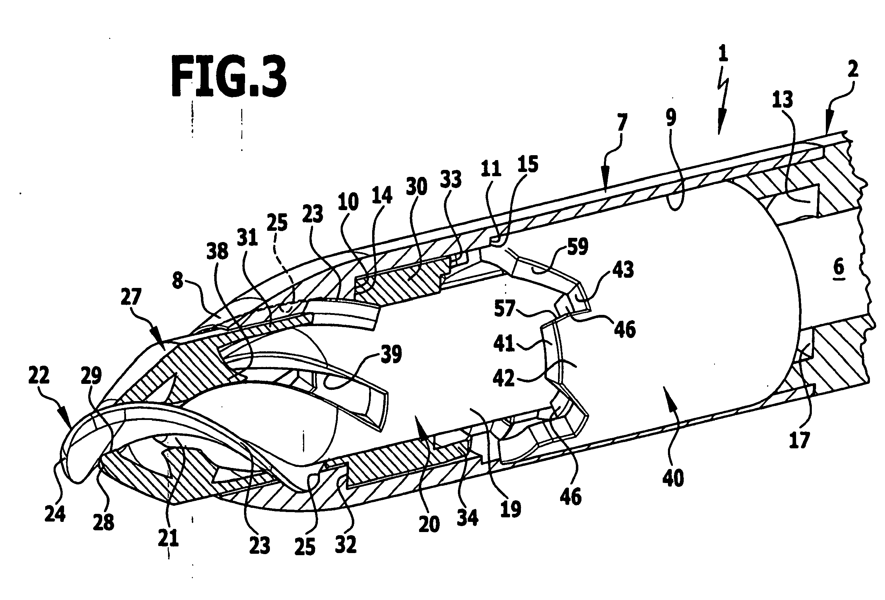

- Figur 3:: eine Ansicht ähnlich Figur 2, bei der zusätzlich die Schutzkappe in Längsrichtung geschnitten ist;

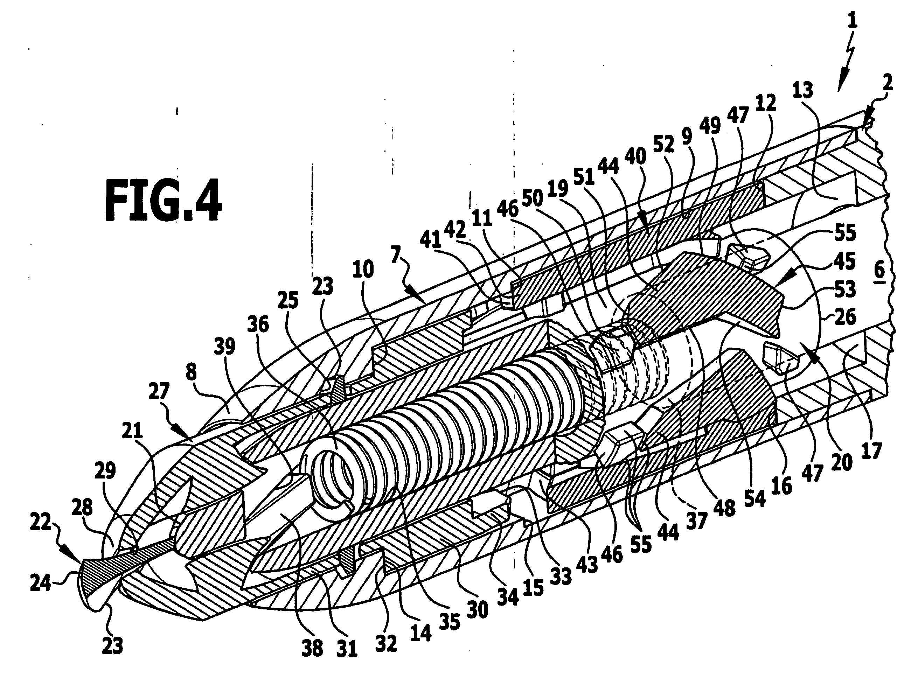

- Figur 4:: eine Ansicht ähnlich Figur 3, bei der zusätzlich das Schaltelement und teilweise der Messerträger in Längsrichtung geschnitten sind;
- Figur 5:: eine Explosionsdarstellung des vorderen Teils des rohrförmigen Gehäuses, des Messerträgers, der Schutzkappe und des Schalt- elementes;
- Figur 6:: eine Längsschnittansicht des Obturators der Figur 1 in der Aus- gangsposition mit vorgeschobener Schutzkappe und zurückge- schobenem Messerträger;
- Figur 7:: eine Ansicht ähnlich-Figur 6 mit über die Schneidstellung nach vorne vorgeschobenem Messerträger;
- Figur 8:: eine Ansicht ähnlich Figur 7 mit dem Messerträger und dem Schaltelement in Blockierposition;
- Figur 9:: eine Ansicht ähnlich Figur 8 mit dem Messerträger beim Übergang von der Blockierposition in die Schneidstellung;
- Figur 10:: eine Ansicht ähnlich Figur 9 mit dem Messerträger in Schneidstel- lung und der Schutzkappe in Arbeitsstellung;
- Figur 11:: eine Ansicht ähnlich Figur 10 mit der Schutzkappe in Ruhestellung und bei der Rückzugbewegung des Messerträgers;
- Figur 12:: eine schematische Darstellung der Nockenführung zwischen Mes- serträger und Schaltelement bei der Vorschubbewegung des Mes- serträgers von der Schutzstellung in Richtung auf die Schneidstel- lung und bis zu einer über die Schneidstellung hinausgehenden Endstellung;
- Figur 13:: eine Ansicht der Nockenführung zwischen Schaltelement und Messerträger sowie zwischen Schaltelement und Schutzkappe bei der Verschiebung des Messerträgers aus der Endstellung in eine Blockierposition und anschließend in Richtung auf die Schneidstel- lung und
- Figur 14:: eine Ansicht ähnlich Figur 13 bei der Verschiebung des Messer- trägers in die Schneidstellung und anschließend daran von der Schneidstellung zurück in die Schutzstellung und damit in die Ausgangsposition des Schaltelementes.

Der in der Zeichnung dargestellte chirurgische Obturator 1 umfasst ein längliches, rohrförmiges Gehäuse 2, an dessen hinterem Ende 3 ein Griff 4 mit vergrößertem Durchmesser angeordnet ist. In dem Griff 4 ist ein Druckknopf 5 in Längsrichtung des Gehäuses 2 verschieblich gelagert, der von der Rückseite des Griffes 4 betätigt werden kann und der mit einer Schubstange 6 verbunden ist, die im Inneren des rohrförmigen Gehäuses 2 bis in dessen vorderen Bereich A führt.

In diesem vorderen Bereich A endet das rohrförmige Gehäuse 2 in einer Teil des rohrförmigen Gehäuses 2 bildenden im wesentlichen kreiszylindrischen und zu ihrem vorderen Ende 8 hin sich verjüngenden Endkappe 7, die an ihrem vorderen Ende 8 offen ist.

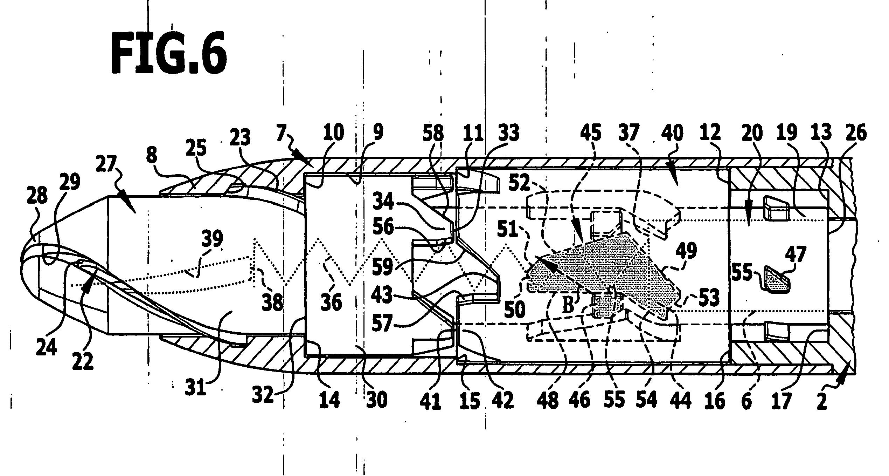

Die Endkappe 7 umschließt einen Innenraum 9, der vom vorderen Ende 8 ausgehend zwei aufeinanderfolgende stufenförmige Erweiterungen 10, 11 und anschließend zwei aufeinanderfolgende stufenförmige Verengungen 12, 13 aufweist, so dass an diesen stufenförmigen Erweiterungen und Verengungen jeweils ringschulterförmige Vorsprünge 14, 15, 16, 17 entstehen (Figur 6).

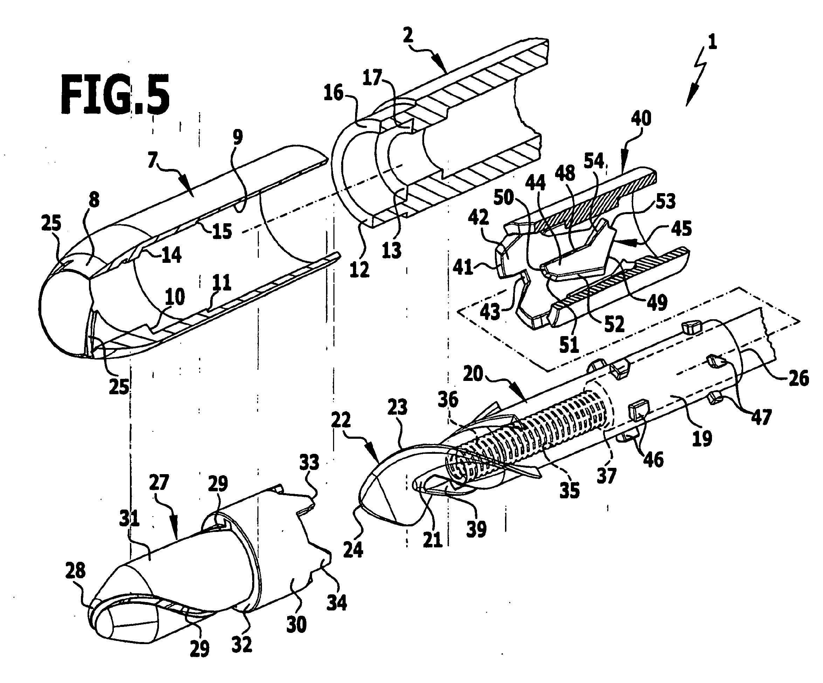

Die Schubstange 6 ist fest verbunden mit einem zylindrischen Schaft 19 eines Messerträgers 20, der den gesamten Innenraum 9 durchsetzt und an seinem vorderen Ende 21 ein über dieses vorstehendes Messer 22 mit einer schraubenlinienförmigen Schneidkante 23 trägt, die von einer Spitze 24 aus nach zwei gegenüberliegenden Seiten des Messerträgers 20 nach hinten verläuft (Figur 5). Die Schneidkante 23 greift in eine entsprechende Führung 25 in der Innenwand der Endkappe 7 ein, diese Führung 25 wird durch eine schraubenlinienförmige Nut in der Innenwand der Endkappe 7 gebildet. Dadurch erfährt der Messerträger 20 beim Vorschieben durch die Schubstange 6 eine zusätzliche Drehbewegung um die Längsachse des rohrförmigen Gehäuses 2.

In einer zurückgezogenen Schutzstellung des Messerträgers 20 liegt dieser mit seinem hinteren, radial über den Umfang der Schubstange 6 vorstehenden Rand 26 an dem Vorsprung 17 des Innenraumes 9 an, damit ist die am weitesten zurückgezogene Stellung des Messerträgers 20 definiert. Von dieser Stellung ausgehend kann der Messerträger 20 mit Hilfe der Schubstange 6 nach vorne geschoben werden in eine Schneidstellung und noch darüber hinaus, in allen Fällen ragt der vordere Bereich des Messers 22 dabei über die Endkappe 7 des rohrförmigen Gehäuses 2 hervor.

Der das Messer 22 tragende vordere Bereich des Messerträgers 20 wird überfangen von einer Schutzkappe 27, die zwischen der Endkappe 7 und dem Messerträger 20 angeordnet ist und diesen allseits umgibt. Die Schutzkappe 27 ist an ihrem vorderen Ende 28 geschlossen und verjüngt sich zu diesem vorderen Ende 28 hin, sie weist einen schraubenförmigen Schlitz 29 auf, dessen Kontur der schraubenförmigen Schneidkante 23 entspricht, die durch diesen Schlitz 29 hindurchragt. Durch diesen Schlitz 29 und die durch diesen hindurchragende Schneidkante 23, die in der Führung 25 der Endkappe 7 geführt ist, erfährt auch die Schutzkappe 27 bei einer Längsverschiebung eine Drehung, die dem schraubenförmigen Verlauf der Schneidkante 23 entspricht.

Die Schutzkappe 27 trägt an ihrem hinteren Ende eine ringflanschförmige Erweiterung 30, die im Übergang zu dem vorderen, das Messer 22 überfangenden zylindrischen Bereich 31 eine Ringstufe 32 ausbildet und die an ihrer hinteren Kante 33 mehrere über den Umfang gleichmäßig verteilte und in Richtung auf das hintere Ende 3 des Gehäuses 2 weisende Zähne 34 trägt. Die Schutzkappe 27 ist sowohl in ihrem vorderen Bereich 31 als auch im Bereich der Erweiterung 30 in einem ringförmigen Zwischenraum zwischen dem Messerträger 20 und der Endkappe 7 frei verdrehbar und in Längsrichtung verschieblich gelagert, sie umgibt dabei den Messerträger 20 konzentrisch.

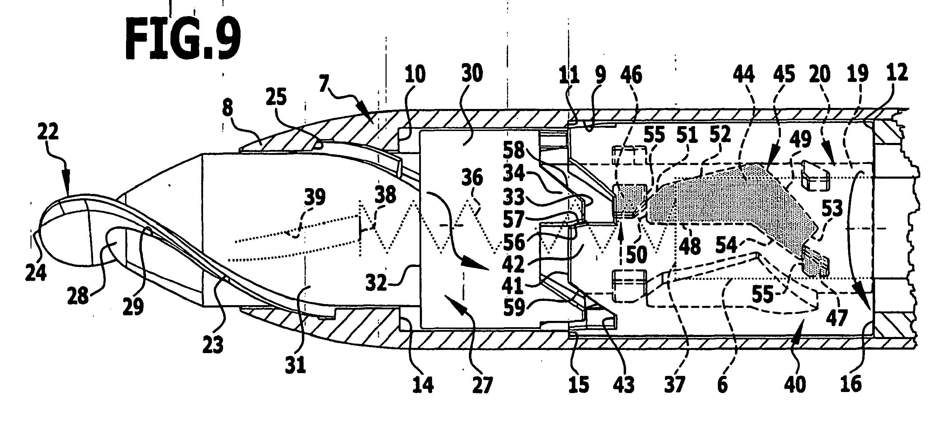

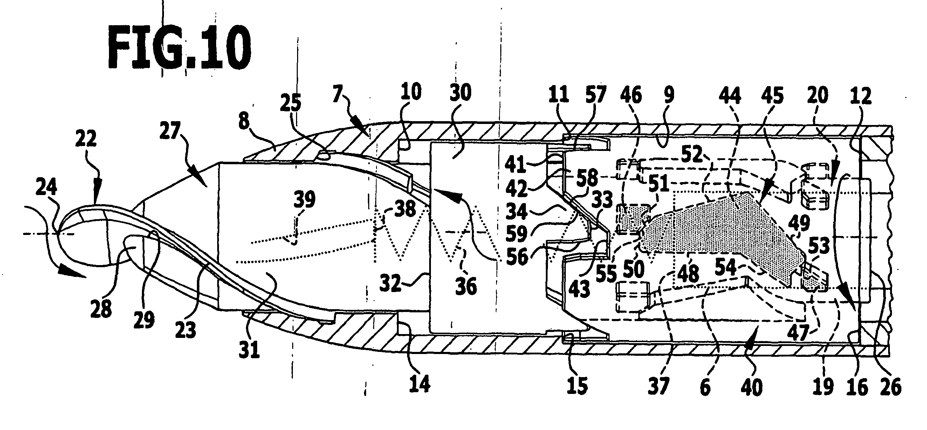

In einer vorgeschobenen Ruhestellung liegt die Ringstufe 32 der Schutzkappe 27 am Vorsprung 14 der Endkappe 7 an, ausgehend von dieser Ruhestellung kann die Schutzkappe 27 in die Endkappe 7 hineingezogen werden bis zu einer zurückgeschobenen Arbeitsstellung, in der die in der Ruhestellung vor dem Vorsprung 15 endenden Zähne 34 über diesen Vorsprung 15 hervorstehen (Figuren 9 und 10). Der Verschiebeweg ist dabei gering und beschränkt sich auf einen Teil der Tiefe der Zähne 34.

Im Innenraum 35 des Messerträgers 20 ist eine Schraubenfeder 36 konzentrisch zu dem Messerträger 20 und der Schutzkappe 27 angeordnet, die sich einerseits an der Stirnfläche 37 der Schubstange 6 und andererseits an einem Vorsprung 38 abstützt, der Teil der Schutzkappe 27 ist und der durch eine schlitzförmige Öffnung 39 in der Wand des Messerträgers 20 in dessen Innenraum 35 hineinragt. Durch diese Schraubenfeder 36 werden die Schutzkappe 27 in ihre Ruhestellung und der Messerträger 20 in seine Schutzstellung verschoben, d.h. die Schutzkappe 27 liegt mit ihrer Ringstufe 32 am Vorsprung 14 an und der Messerträger 20 mit seinem hinteren Rand 26 am Vorsprung 17. Gegen die Kraft der Schraubenfeder 36 kann der Messerträger 20 in Richtung auf die Schneidstellung verschoben werden, ebenso ist eine Verschiebung der Schutzkappe 27 in Richtung auf die Arbeitsstellung möglich.

In einem ringförmigen Zwischenraum zwischen dem Schaft 19 des Messerträgers 20 und der Innenwand der Endkappe 7 ist ein den Schaft 19 konzentrisch umgebendes, hülsenförmiges Schaltelement 40 frei drehbar gelagert, welches sowohl an dem Vorsprung 15 als auch an dem Vorsprung 16 anliegt und dadurch in axialer Richtung unverschieblich in der Endkappe 7 gelagert ist. Dieses Schaltelement 40 trägt an seinem vorderen, der Schutzkappe 27 zugewandten Rand 41 den Zähnen 34 der Schutzkappe 27 gegenüberliegende, über den Umfang gleichmäßig verteilte Zähne 42, die durch vom Rand 41 ausgehende Einschnitte 43 gebildet werden.

An der Innenseite des Schaltelementes 40 befinden sich über den Umfang gleichmäßig verteilt mehrere inselförmige Vorsprünge 44, deren äußerer Rand eine Nockenbahn 45 für Nocken 46, 47 bildet, die am Schaft 19 des Messerträgers 20 angeordnet sind und von diesem radial nach außen abstehen. Dabei trägt der Schaft 19 mehrere gleichmäßig über den Umfang verteilte vordere Nocken 46 sowie mehrere ebenfalls gleichmäßig über den Umfang verteilte hintere Nocken 47, die sich jeweils in einer Radialebene befinden (Figur 5). Diese Nocken 46 und 47 können bei der Verschiebung des Messerträgers 20 in Längsrichtung durch Anlage an den Nockenbahnen 45 das Schaltelement 40 verdrehen, wobei die vorderen und die hinteren Nocken nacheinander an geeigneten Abschnitten der Nockenbahn 45 zur Anlage kommen und die Drehung entsprechend der Form und Anordnung dieser Abschnitte der Nockenbahn 45 durchführen.

Die in sich geschlossene Nockenbahn 45 des dargestellten Ausführungsbeispiels weist einen ersten, unteren, parallel zur Längsrichtung des Gehäuses 2 verlaufenden Abschnitt 48, einen sich an der Vorderseite des inselförmigen Vorsprunges 44 anschließenden, quer zur Längsrichtung des Gehäuses 2 verlaufenden dritten Abschnitt 50, einen sich daran anschließenden, etwa unter 45 ° zur Längsachse des Gehäuses 2 nach hinten geneigten vierten Abschnitt 51, einen sich daran anschließenden, jedoch weniger stark gegenüber der Längsrichtung des Gehäuses 2 geneigten fünften Abschnitt 52 sowie einen von dessen Ende in entgegengesetzter Richtung etwa unter 45 ° relativ zur Längsachse des Gehäuses 2 geneigten zweiten Abschnitt 49 auf. Am hinteren Ende des zweiten Abschnittes 49 schließt sich ein sechster Abschnitt 53 an mit einem oberen Teil, der vom Ende des zweiten Abschnittes 49 etwa unter einem rechten Winkel nach vorne verläuft, und einem unteren Teil, der quer zur Längsrichtung des Gehäuses 2 angeordnet ist, und das untere Ende des sechsten Abschnittes 53 ist über einen siebten Abschnitt 54 mit dem hinteren Ende des ersten Abschnittes-48-verbunden.. Alle Abschnitte des dargestellten Ausführungsbeispiels sind in sich geradlinig ausgebildet.

Die Nocken 46 und 47 sind so geformt, dass sie an den Seiten, an denen sie an unterschiedlichen Abschnitten der Nockenbahn 45 anliegen, jeweils parallel zu diesen Abschnitten verlaufende Anlageflächen 55 aufweisen, d.h. die Aussenkontur der Nocken ergibt sich auf diese Weise einmal durch die Orientierung der Abschnitte 48 bis 54 und zum anderen aus der Anlage oder Nichtanlage des jeweiligen Nockens an der Nockenbahn im Bereich entsprechender Abschnitte. Da vordere und hintere Nocken vorgesehen sind, liegt nicht jeder Nocken zwangsläufig an allen Abschnitten der Nockenbahn an, sondern die Anlage wird von den vorderen und hinteren Nocken nacheinander übernommen.

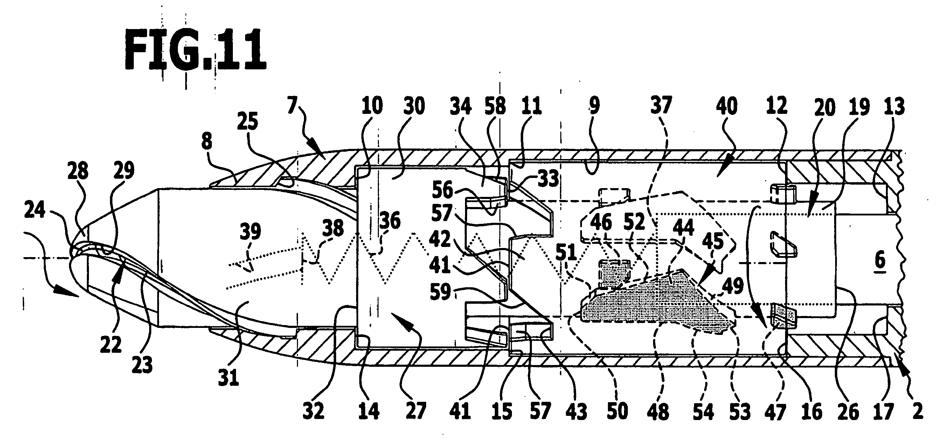

Zur Funktion der beschriebenen Anordnung wird nachstehend insbesondere auf die Darstellungen der Figuren 6 bis 14 Bezug genommen. Vor der Betätigung befindet sich die gesamte Anordnung in einer in Figur 6 dargestellten Ausgangsposition, bei welcher die Schutzkappe 27 in ihrer vorgeschobenen Ruhestellung und der Messerträger 20 in seiner zurückgezogenen Schutzstellung stehen. Die Zähne 34 der Schutzkappe 27 sind außer Eingriff mit den Zähnen 42 des hülsenförmigen Schaltelementes 40. Die Schneidkante 23 des Messers 22 ist dabei in dem Bereich, in dem die Schutzkappe 27 über die Endkappe 7 vorsteht, vollständig in das Innere der Schutzkappe 27 zurückgezogen, so dass die Schneidkante 23 außerhalb der Endkappe 7 nicht über die Schutzkappe 27 vorsteht. Allerdings tritt die Schneidkante 23 in dem im Inneren der Endkappe 7 angeordneten Teil der Schutzkappe 27 weiterhin durch den Schlitz 29 der Schutzkappe 27 hindurch in die Führung 25 in der Innenwand der Endkappe 7. In dieser Stellung werden der Messerträger 20 und die Schutzkappe 27 durch die Schraubenfeder 36 gehalten, die diese beiden Teile auseinanderdrückt.

In dieser Ausgangsposition liegen die vorderen Nocken 46 jeweils am hinteren Ende des ersten Abschnitts 48 der Nockenbahn 45 an, die hinteren Nocken 47 sind gegenüber den Nockenbahnen 45 nach hinten zurückgezogen und stehen mit diesen nicht in Kontakt.

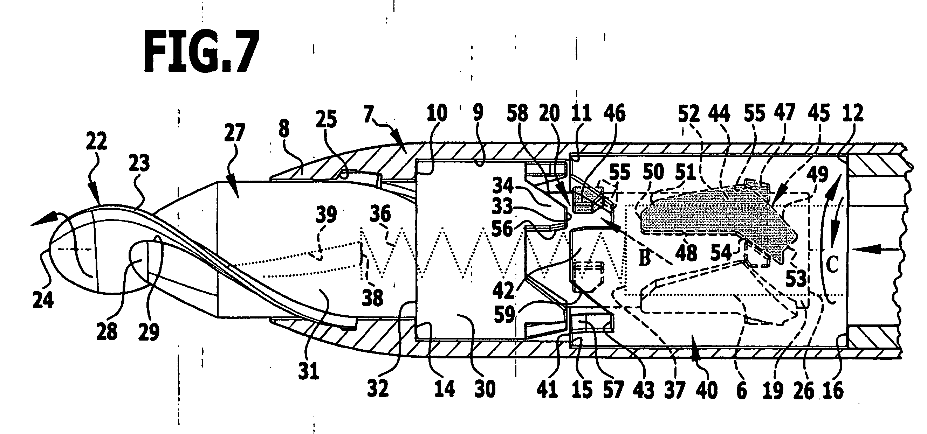

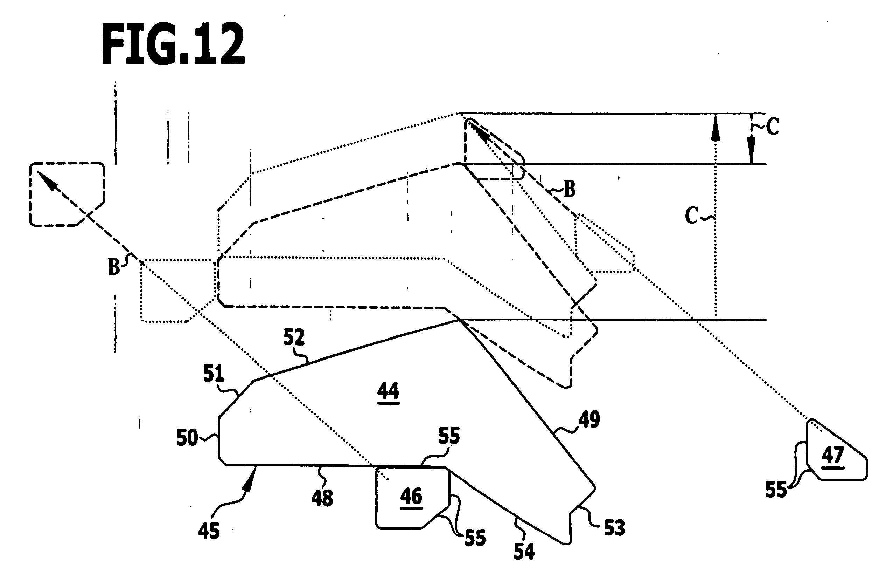

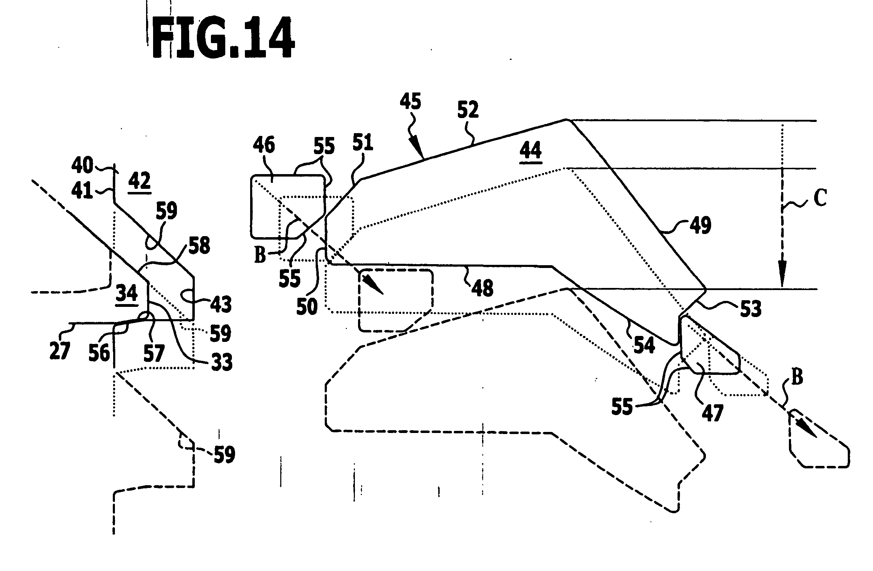

Für den Einsatz des Obturators ist es zunächst notwendig, das Messer zu aktivieren, dies erfolgt durch einen Druck auf den Druckknopf 5 und damit durch ein Vorschieben der Schubstange 6 und des fest damit verbundenen Messerträgers 20. Dieser wird entgegen der Wirkung der Schraubenfeder 36 vorgeschoben und dabei durch die Führung der schraubenförmigen Schneidkante 23 in der Führung 25 auch geringfügig verdreht. Der vordere Nocken 46 bewegt sich dadurch auf einer Bahn, die schräg zur Längsrichtung des Gehäuses 2 verläuft, in den Figuren 6 und 14 ist diese Bahn jeweils durch den Pfeil B angedeutet. Bei dieser Verschiebung gleiten die vorderen Nocken 46 jeweils am ersten Abschnitt 48 der Nockenbahn 45 entlang und verdrehen dabei das hülsenförmige Schaltelement 40 in Richtung der Pfeile C in den Figuren 7 und 12 in einer ersten Richtung. Die Nockenbahn 45 wird also bei dieser Verdrehung bei der Darstellung der Figuren 7 und 12 nach oben verschoben.

Bei der Vorschubbewegung des Messerträgers 20 gelangen die vorderen Nocken 46 vom hinteren Ende des ersten Abschnittes 48 (ausgezogene Kontur in Figur 12) an das vordere Ende des ersten Abschnittes 48 (gepunktete Kontur in Figur 12), und anschließend werden sie noch weiter nach vorne vorgeschoben in eine Endstellung (gestrichelte Kontur in Figur 12). Diese weitere Verschiebung erfolgt für die vorderen Nocken 46 ohne Anlage an der Nockenbahn -45, d.h. die vorderen Nocken 46 verdrehen das Schaltelement 40 nur während ihrer Anlage am ersten Abschnitt 48.

Die Anordnung der vorderen Nocken 46 und der hinteren Nocken 47 ist so gewählt, dass die hinteren Nocken 47 zur Anlage am zweiten Abschnitt 49 gelangen, sobald die vorderen Nocken 46 den ersten Abschnitt 48 verlassen haben. Auch die hinteren Nocken 47 werden auf einer schräg zur Längsachse geneigten Bahn verschoben, die ebenfalls der Richtung der Pfeile B entspricht. Da der zweite Abschnitt 49 stärker gegenüber der Längsrichtung des Gehäuses 2 geneigt ist als diese durch den Pfeil B gekennzeichnete Bewegungsrichtung der hinteren Nocken 47, legen sich die hinteren Nocken 47 an den zweiten Abschnitt 49 an und verdrehen bei der weiteren Verschiebung des Messerträgers 20 das Schaltelement 40 entgegen der ersten Richtung in einer zweiten Richtung, dies ist in Figur 12 ebenfalls durch den Pfeil C angedeutet.

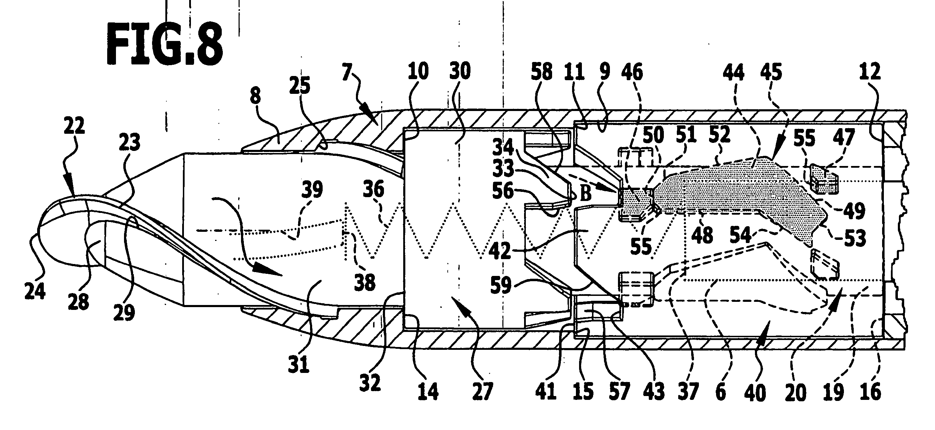

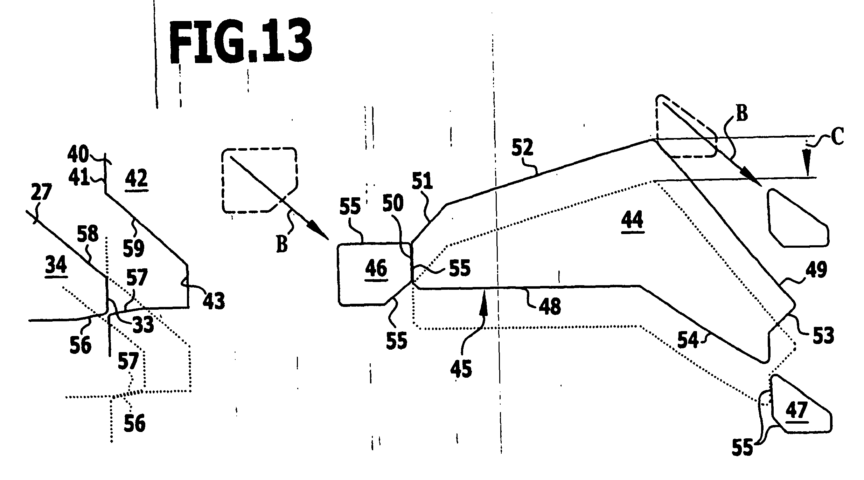

Die Vorschubbewegung des Messerträgers 20 wird durch einen in der Zeichnung nicht dargestellten Anschlag begrenzt, beispielsweise kann dieser Anschlag am Druckknopf 5 vorgesehen sein. Lässt der Benutzer nach Erreichen dieses vordersten Punktes den Druckknopf wieder los, wird der Messerträger 20 unter der Wirkung der Schraubenfeder 36 zurückgeschoben, und zwar auf demselben durch den Pfeil B angedeuteten Weg. Dabei treffen die vorderen Nocken 46 auf den dritten Abschnitt der Nockenbahn 45, da die Nockenbahn durch den hinteren Nocken 47 in der zweiten Richtung verdreht worden ist und jetzt mit dem dritten Abschnitt 50 im Verschiebeweg der vorderen Nocken 46 liegt (Figur 13). Die Rückbewegung des Messerträgers 20 wird also blockiert durch die Anlage der vorderen Nocken 46 am dritten Abschnitt 50 der Nockenbahn 45. In dieser Blockierposition ragt die Schneidkante 23 im gesamten Bereich des Schlitzes 29 aus der Schutzkappe 27 heraus, wie dies in Figur 8 dargestellt ist, der Obturator ist in dieser Blockierposition bereit für den Einsatz zum Durchstechen einer Körperwand (ausgezogene Kontur in Figur 13).

Bei dem Durchstechen setzt der Operateur die Spitze 24 des Messers 22 auf die Körperwand und schiebt den Obturator unter gleichzeitiger Drehung in Richtung auf die Körperwand vor, so dass das Messer 22 längs der Schneidkante 23 in die Körperwand eindringt. Bei diesem Eindringen legt sich das vordere Ende 28 der Schutzkappe 27 an die Körperwand an und wird von dieser entgegen der Wirkung der Schraubenfeder 36 unter gleichzeitiger Verdrehung durch die Führung der schraubenförmigen Schneidkante 23 in die Endkappe 7 hineingeschoben (Figuren 9 und 13). Dabei tauchen die Zähne 34 der Schutzkappe 27 zwischen die Zähne 42 des Schaltelementes 40 ein und liegen dabei mit jeweils einer sehr steilen, gegenüber der Längsrichtung des Gehäuses 2 nur geringfügig geneigten Flanke 56 bzw. 57 aneinander an. Dies führt zu einer Verdrehung des Schaltelementes 40 in der zweiten Richtung, so dass dadurch die vorderen Nocken 46 des Messerträgers 20 bis über das Ende des dritten Abschnittes 50 hinaus verschoben werden (gepunktete Kontur in Figur 13 und ausgezogene Kontur in Figur 14). Die vorderen Nocken 46 gelangen somit an den Beginn des schräg nach hinten verlaufenden vierten Abschnittes 51, dies führt zu einer geringfügigen Rückbewegung des Messerträgers 20 und zu einer weiteren Verdrehung des Schaltelementes 40 in der zweiten Richtung (gepunktete Kontur in Figur 14).

Allerdings sind sowohl diese Rückbewegung des Messerträgers 20 als auch die Verdrehung des Schaltelementes 40 sehr geringfügig, da bei der Verdrehung des Schaltelementes 40 die gegenüberliegenden Flanken 58 bzw. 59 der Zähne 34 und 42 zur Anlage kommen und eine weitere Drehbewegung des Schaltelementes 40 verhindern (Figuren 10 und 14). In dieser Stellung ist der Messerträger 20 daran gehindert, weiter nach hinten verschoben zu werden, da sich die vorderen Nocken 46 auf dem vierten Abschnitt 51 der Nockenbahn 45 abstützen (gepunktete Stellung in Figur 14). Diese Stellung wird als Schneidstellung des Messerträgers 20 bezeichnet, bei dieser Schneidstellung steht die Schneidkante 23 weiterhin über die Schutzkappe 27 hervor, die sich ihrerseits in der zurückgezogenen Arbeitsstellung befindet. Der Operateur kann also das Durchstechen der Körperwand fortsetzen, bis diese vollständig durchstochen

Dies führt dazu, dass beim weiteren Vorschieben des Obturators 1 auch die Schutzkappe 27 mit ihrem sich verjüngenden vorderen Ende 28 und mit dem sich daran anschließenden zylindrischen Bereich 31 durch die in die Körperwand eingebrachte Öffnung hindurchtritt und nun nicht mehr genügend kräftig gegen die Wirkung der Schraubenfeder 36 in die Endkappe 7 eingeschoben wird, sondern die Schutzkappe 27 kann nunmehr unter der Wirkung der Schraubenfeder 36 wieder nach vorne in ihre Ruhestellung verschoben werden, bis die Ringstufe 32 der schaftförmigen Erweiterung 30 am Vorsprung 14 anliegt. Dadurch kommen die Zähne 34 der Schutzkappe 27 außer Eingriff mit den Zähnen 42, d.h. dadurch wird das Schaltelement 40 freigegeben und kann sich nunmehr wieder frei drehen. Unter der Wirkung der Schraubenfeder 36 wird daher jetzt der Messerträger 20 in seine Schutzstellung zurückgeschoben, da das Schaltelement 40 diese Rückbewegung nicht mehr blockiert. Das Schaltelement 40 wird dabei gleichzeitig in der zweiten Richtung verdreht, da die vorderen Nocken 46 am fünften Abschnitt 52 entlang (gestrichelte Kontur in Figur 14) gleiten, bis wieder die in Figur 6 dargestellte Ausgangsposition erreicht ist, bei der der Messerträger 20 vollständig zurückgezogen ist und bei der die Schneidkante 23 in dem außerhalb der Endkappe 7 liegenden Teil vollständig von der Schutzkappe 27 überfangen wird.

Damit ist ein voller Arbeitszyklus durchlaufen worden, der in gleicher Weise wiederholt werden kann.

Aus der Darstellung der Figur 14 wird deutlich, dass die hinteren Nocken 47 dem sechsten Abschnitt 53 der Nockenbahn 45 gegenüberstehen, wenn sich der Messerträger 20 von der Blockierposition in die Schneidstellung bewegt (gepunktete Kontur in Figur 13 und ausgezogene sowie gepunktete Konturen in Figur 14). Dadurch wird ein Vorschieben des Messerträgers 20 in Richtung auf die Endstellung, also über die Blockier- bzw. Schneidstellung hinaus, verhindert. Dies stellt eine Sicherung dar für den Fall, dass der Operateur bei dem Schneidvorgang ungewollt den Druckknopf 5 betätigen sollte. Diese Betätigung-führt zu keiner Verschiebung des Messerträgers 20, da dieser in beiden Richtungen gegen eine axiale Verschiebung gesichert ist, und zwar durch die an gegenüberliegenden Seiten an der Nockenbahn 45 anliegenden vorderen Nocken 46 und hinteren Nocken 47.

Der Aufbau der beschriebenen Anordnung ist sehr einfach, da lediglich in dem rohrförmigen Gehäuse drei bewegliche Teile und eine Schraubenfeder eingesetzt werden müssen, auch der Zusammenbau ist sehr einfach, da es genügt, diese Teile konzentrisch zusammenzustecken und die Endkappe dann fest mit dem anschließenden schaftförmigen Teil des rohrförmigen Gehäuses zu verbinden. In gleicher Weise kann auch eine Trennung und damit eine Reinigung erfolgen.

Die gesamte Anordnung ist im messernahen vorderen Teil des rohrförmigen Gehäuses unterzubringen, ohne dass dadurch dessen Außendurchmesser vergrößert werden muss, so dass im Griffbereich des Obturators 1 mit Ausnahme des Druckknopfes 5 keinerlei Teile der Rückzugseinrichtung angeordnet werden müssen.

## Patentansprüche

1. Chirurgischer Obturator (1) zum Durchstechen einer Körperwand mit einem rohrförmigen Gehäuse (2), mit einem in dem rohrförmigen Gehäuse (2) in Längsrichtung zwischen einer vorgeschobenen Schneidstellung und einer zurückgezogenen Schutzstellung verschieblichen Messerträger (20), an dem ein Messer (22) mit einer Schneidkante (23) gehalten ist, die in einer entsprechend verlaufenden Führung des rohrförmigen Gehäuses (2) geführt ist, mit einer das Messer (22) überfangenden, im rohrförmigen Gehäuse (2) in Längsrichtung zwischen einer vorgeschobenen Ruhestellung, in der das Messer (22) in seiner Schutzstellung vollständig überdeckt ist, und einer zurückgezogenen Arbeitsstellung verschiebbaren Schutzkappe (27), die einen Schlitz (29)aufweist, durch welchen die Schneidkante (23) des Messers (22) hindurchtritt und in der Arbeitsstellung der Schutzkappe (27) über diese hervorsteht, und mit einer in dem rohrförmigen Gehäuse (2) angeordneten, das Messer (22) aus der Schneidstellung in die Schutzstellung verschiebenden Rückzugseinrichtung, die durch Verschiebung der Schutzkappe (27) von der Arbeitsstellung in die Ruhestellung aktivierbar ist, **dadurch gekennzeichnet, dass** die Rückzugseinrichtung ein das Messer (22) in der Schneidstellung fixierendes und bei Aktivierung für eine Verschiebung in die Ruhestellung freigebendes, um die Längsachse des rohrförmigen Gehäuses (2) frei drehbar und in Längsrichtung unverschieblich im rohrförmigen Gehäuse (2) gelagertes Schaltelement (40) aufweist, welches bei einer Verschiebung des Messerträgers (20) und/oder der Schutzkappe (27) in Längsrichtung des rohrförmigen Gehäuses (2) über eine zwischen dem Schaltelement (40) einerseits und dem Messerträger (20) und/oder der Schutzkappe (27) andererseits wirksame Nockenführung (45, 46, 47; 34, 42) verdrehbar ist, und dass die Schutzkappe (27) und der Messerträger (20) von einer diese auseinander schiebenden gemeinsamen Feder (36) beaufschlagt sind, welche die Schutzkappe (27) von der Arbeitsstellung in die Ruhestellung und den Messerträger (20) von der Schneidstellung in die Schutzstellung verschiebt.

2. Chirurgischer Obturator nach Anspruch 1, **dadurch gekennzeichnet, dass** die Feder (36) eine im Inneren des Messerträgers (20) angeordnete Schraubenfeder ist, die sich an einem Ende an einem durch die Wand des Messerträgers (20) in dessen Innenraum (35) eintretenden Vorsprung (38) der Schutzkappe (27) abstützt.

3. Chirurgischer Obturator nach Anspruch 1 oder 2, **dadurch gekennzeichnet, dass** die Schneidkante (23) des Messers (22) schraubenlinienförmig ist, so dass der Messerträger (20) bei Verschiebung in Längsrichtung relativ zum rohrförmigen Gehäuse (2) und die Schutzkappe (27) bei einer Längsverschiebung relativ zum Messerträger (20) relativ zueinander um die Längsachse des Messerträgers (20) verdreht werden.

4. Chirurgischer Obturator nach einem der voranstehenden Ansprüche,
**dadurch gekennzeichnet, dass** der Messerträger (20) einen hülsenförmigen Schaft (19) aufweist, der konzentrisch zum rohrförmigen Gehäuse (2) angeordnet ist.

5. Chirurgischer Obturator nach einem der voranstehenden Ansprüche,
**dadurch gekennzeichnet, dass** der Messerträger (20) mit einer Schubstange (6) verbunden ist, die durch das rohrförmige Gehäuse (2) bis zu einer Druckeinrichtung (5) an dem dem Messer (22) gegenüberliegenden Ende des rohrförmigen Gehäuses (2) verläuft.

6. Chirurgischer Obturator nach einem der voranstehenden Ansprüche,
**dadurch gekennzeichnet, dass** das Schaltelement (40) eine den Messerträger (20) konzentrisch umgebende Hülse ist.

7. Chirurgischer Obturator nach Anspruch 6, **dadurch gekennzeichnet, dass** die zwischen dem Schaltelement (40) und dem Messerträger (20) wirksame Nockenführung durch Nockenbahnen (45) und daran anlegbare Nocken (46, 47) gebildet werden, die an der Außenseite des Messerträgers (20) bzw. der Innenwand des hülsenförmigen Schaltelements (40) angeordnet sind.

8. Chirurgischer Obturator nach Anspruch 7, **dadurch gekennzeichnet, dass** längs des Umfanges des Messerträgers (20) und des hülsenförmigen Schaltelementes (40) mehrere gleichartige Nockenbahnen (45) und Nocken (46, 47) angeordnet sind derart, dass die Nocken (46, 47) bei aufeinanderfolgenden Arbeitszyklen jeweils an einer benachbarten Nockenbahn (45) anliegen.

9. Chirurgischer Obturator nach einem der Ansprüche 7 oder 8, **dadurch gekennzeichnet, dass** jeder Nockenbahn (45) zwei Nocken (46, 47) zugeordnet sind, die beim Verschieben des Messerträgers (20) relativ zu dem Schaltelement (40) nacheinander an der Nockenbahn (45) anliegen.

10. Chirurgischer Obturator nach einem der voranstehenden Ansprüche, **dadurch gekennzeichnet, dass** die Nockenbahn (45) durch den Rand eines inselförmigen Vorsprungs (44) gebildet wird.

11. Chirurgischer Obturator nach einem der Ansprüche 7 bis 10, **dadurch gekennzeichnet, dass** ein dem Messer (22) zugewandter vorderer Nocken (46) beim Verschieben des Messerträgers (20) aus der Schutzstellung in Richtung auf die Schneidstellung an einem schräg zur Verschieberichtung des Messerträgers (20) verlaufenden ersten Abschnitt (48) der Nockenbahn (45) entlanggleitet und **dadurch** das Schaltelement (40) ausgehend von einer Ausgangsposition in einer ersten Richtung verdreht, dass ein dem Messer (22) abgewandter hinterer Nocken (47) an einem zweiten, in der entgegengesetzten Richtung schräg zur Verschieberichtung des Messerträgers (20) verlaufenden Abschnitt (49) zur Anlage gelangt, sobald der vordere Nocken (46) an dem ersten Abschnitt (48) der Nockenbahn (45) vorbeigeschoben ist, so dass das Schaltelement (40) in der entgegengesetzten Richtung in eine Blockierposition verdreht wird, in der beim anschließenden Zurückschieben des Messerträgers (20) aus einer Endstellung, die vor der Schneidstellung liegt, ein an diesem angeordneter Anschlag (46) an einem Anschlag (50) am Schaltelement (40) anstößt und **dadurch** ein weiteres Zurückschieben des Messerträgers (20) verhindert.

12. Chirurgischer Obturator nach Anspruch 11, **dadurch gekennzeichnet, dass** der Anschlag am Messerträger (20) durch den vorderen Nocken (46) gebildet wird.

13. Chirurgischer Obturator nach Anspruch 11 oder 12, **dadurch gekennzeichnet, dass** der Anschlag am Schaltelement (40) durch einen quer zur Längsrichtung des rohrförmigen Gehäuses (2) verlaufenden dritten Abschnitt (50) der Nockenbahn (45) gebildet wird.

14. Chirurgischer Obturator nach einem der Ansprüche 6 bis 13, **dadurch gekennzeichnet, dass** die Nockenbahn und die Nocken zwischen dem Schaltelement (40) und der Schutzkappe (27) durch die seitlichen Flanken (56, 57, 58, 59) von Zähnen (34, 42) gebildet werden, die an den einander zugewandten Stirnseiten der Schutzkappe (27) und des Schaltelementes (40) angeordnet sind und die bei in die Arbeitsstellung zurückgeschobener Schutzkappe (27) in Eingriff stehen.

15. Chirurgischer Obturator nach einem der Ansprüche 11 bis 13 und nach Anspruch 14, **dadurch gekennzeichnet, dass** die Flanken (56, 57) der Zähne (34, 42) so angeordnet und ausgebildet sind, dass die Zähne (34) der Schutzkappe (27) beim Zurückziehen der Schutzkappe (27) von der Ruhestellung in die Arbeitsstellung das Schaltelement (40) aus seiner Blockierposition in der ersten Richtung verdrehen, bis der Anschlag (46) des Messerträgers (20) auf einen schräg zur Längsrichtung des rohrförmigen Gehäuses (2) verlaufenden vierten Abschnitt (51) der Nockenbahn (45) des Schaltelements (40) gelangt, der sich an den Anschlag (50) des Schaltelements (40) anschließt.

16. Chirurgischer Obturator nach Anspruch 15, **dadurch gekennzeichnet, dass** die Flanken (58, 59) der Zähne (34, 42) so ausgebildet sind, dass eine Drehung des Schaltelements (40) in der zweiten Richtung unter dem Einfluss des am vierten Abschnitt (51) anliegenden Anschlages (46) des Messerträgers (20) derart begrenzt wird, dass der Messerträger (20) gegenüber seiner Stellung bei Anlage der beiden Anschläge (46, 50) aneinander nur wenig zurückgeschoben wird bis in seine Schneidstellung.

17. Chirurgischer Obturator nach Anspruch 16, **dadurch gekennzeichnet, dass** der Eingriff der Zähne (34, 42) des Messerträgers (20) und der Schutzkappe (27) bei Verschiebung der Schutzkappe (27) in die Ruhestellung aufgehoben wird, so dass die Begrenzung der Verdrehbarkeit des Schaltelementes (40) aufgehoben wird.

18. Chirurgischer Obturator nach Anspruch 17, **dadurch gekennzeichnet, dass** die Nockenbahn (45) einen sich an den schrägen vierten Abschnitt (51) anschließenden fünften Abschnitt (52) aufweist, an dem der vordere Nocken (46) des Messerträgers (20) beim Zurückschieben des Messerträgers (20) aus der Schneidstellung in die Schutzstellung-entlanggleitet und das Schaltelement (40) dabei in der zweiten Richtung in die Ausgangsposition für den nächsten Arbeitszyklus verdreht.

19. Chirurgischer Obturator nach einem der Ansprüche 11 bis 18, **dadurch gekennzeichnet, dass** der hintere Nocken (47) des Messerträgers (20) bei der Stellung des Schaltelements (40) in der Blockierposition und bei Anlage des Anschlages (46) des Messerträgers (20) an dem vierten Abschnitt (51) einem sechsten Abschnitt (53) der Nockenbahn (45) gegenübersteht, der eine Verschiebung des Messerträgers (20) in Richtung von der Schutzstellung zur Schneidstellung verhindert.

## Claims

1. Surgical obturator (1) for piercing a body wall with a tubular housing (2), with a blade carrier (20) displaceable in the tubular housing (2) in the longitudinal direction between an advanced cutting position and a retracted protected position, a blade (22) with a cutting edge (23) being held on the blade carrier (20), the cutting edge (23) being guided in a correspondingly extending guide of the tubular housing (2), with a protective cap (27) engaging over the blade (22) and being displaceable in the tubular housing (2) in the longitudinal direction between an advanced rest position in which the blade (22) is completely covered in its protected position and a retracted work position, the protective cap (27) having a slit (29) through which the cutting edge (23) of the blade (22) passes and, in the work position of the protective cap (27), projects over the latter, and with a retracting device arranged in the tubular housing (2) and displacing the blade (22) from the cutting position to the protected position, the retracting device being activatable by displacement of the protective cap (27) from the work position to the rest position, **characterized in that** the retracting device comprises a switching element (40) which fixes the blade (22) in the cutting position and, upon activation, releases the blade (22) for displacement to the rest position, the switching element (40) being mounted in the tubular housing (2) so as to be freely rotatable about the longitudinal axis of the tubular housing (2) and immovable in the longitudinal direction, and the switching element (40) being rotatable, upon displacement of the blade carrier (20) and/or the protective cap (27) in the longitudinal direction of the tubular housing (2), by means of a cam guide (45, 46, 47; 34, 42) which is operative between the switching element (40), on the one hand, and the blade carrier (20) and/or the protective cap (27), on the other hand, and **in that** the protective cap (27) and the blade carrier (20) are acted upon by a common spring (36) which pushes these apart, the spring (36) displacing the protective cap (27) from the work position to the rest position and the blade carrier (20) from the cutting position to the protected position.

2. Surgical obturator in accordance with claim 1, **characterized in that** the spring (36) is a helical spring which is arranged in the interior of the blade carrier (20) and is supported at one end on a projection (38) of the protective cap (27) that extends through the wall of the blade carrier (20) into the interior (35) thereof.

3. Surgical obturator in accordance with claim 1 or 2, **characterized in that** the cutting edge (23) of the blade (22) is of helical configuration, so that the blade carrier (20), upon displacement in the longitudinal direction relative to the tubular housing (2), and the protective cap (27), upon longitudinal displacement relative to the blade carrier (20), are rotated relative to each other about the longitudinal axis of the blade carrier (20).

4. Surgical obturator in accordance with any one of the preceding claims, **characterized in that** the blade carrier (20) comprises a sleeve-shaped shaft (19) which is arranged concentrically with the tubular housing (2).

5. Surgical obturator in accordance with any one of the preceding claims, **characterized in that** the blade carrier (20) is connected to a push rod (6) which extends through the tubular housing (2) as far as a push device (5) at the end of the tubular housing (2) remote from the blade (22).

6. Surgical obturator in accordance with any one of the preceding claims, **characterized in that** the switching element (40) is a sleeve which concentrically surrounds the blade carrier (20).

7. Surgical obturator in accordance with claim 6, **characterized in that** the cam guide operative between the switching element (40) and the blade carrier (20) is formed by cam tracks (45) and cams (46, 47) adapted to abut thereon, the cams (46, 47) and the cam tracks (45) being arranged on the outer side of the blade carrier (20) and on the inner wall of the sleeve-shaped switching element (40), respectively.

8. Surgical obturator in accordance with claim 7, **characterized in that** a plurality of similar cam tracks (45) and cams (46, 47) are arranged along the circumference of the blade carrier (20) and the sleeve-shaped switching element (40) such that in successive work cycles the cams (46, 47) respectively abut on an adjacent cam track (45).

9. Surgical obturator in accordance with claim 7 or 8, **characterized in that** each cam track (45) has associated with it two cams (46, 47) which, upon displacement of the blade carrier (20) relative to the switching element (40), abut one after the other on the cam track (45).

10. Surgical obturator in accordance with any one of the preceding claims, **characterized in that** the cam track (45) is formed by the edge of an island-shaped projection (44).

11. Surgical obturator in accordance with any one of claims 7 to 10, **characterized in that** a front cam (46) facing the blade (22), upon displacement of the blade carrier (20) from the protected position in the direction towards the cutting position, slides along a first section (48) of the cam track (45) that extends at an incline to the direction of displacement of the blade carrier (20) and thereby rotates the switching element (40) from an initial position in a first direction, **in that** a rear cam (47) facing away from the blade (22) enters into abutment with a second section (49) extending in the opposite direction at an incline to the direction of displacement of the blade carrier (20) once the front cam (46) has moved past the first section (48) of the cam track (45), so that the switching element (40) is rotated in the opposite direction into a blocking position in which, upon subsequent retraction of the blade carrier (20) from an end position located in front of the cutting position, a stop (46) arranged on the blade carrier (20) strikes a stop (50) on the switching element (40) and thereby prevents any further retraction of the blade carrier (20).

12. Surgical obturator in accordance with claim 11, **characterized in that** the stop on the blade carrier (20) is formed by the front cam (46).

13. Surgical obturator in accordance with claim 11 or 12, **characterized in that** the stop on the switching element (40) is formed by a third section (50) of the cam track (45) that extends transversely to the longitudinal direction of the tubular housing (2).

14. Surgical obturator in accordance with any one of claims 6 to 13, **characterized in that** the cam track and the cams between the switching element (40) and the protective cap (27) are formed by the side flanks (56, 57, 58, 59) of teeth (34, 42) which are arranged on the end faces of the protective cap (27) and the switching element (40) that face one another, and which are in engagement when the protective cap (27) is retracted into the work position.

15. Surgical obturator in accordance with any one of claims 11 to 13 and in accordance with claim 14, **characterized in that** the flanks (56, 57) of the teeth (34, 42) are so arranged and designed that the teeth (34) of the protective cap (27), upon retraction of the protective cap (27) from the rest position to the work position, rotate the switching element (40) from its blocking position in the first direction until the stop (46) of the blade carrier (20) reaches a fourth section (51) of the cam track (45) of the switching element (40), which extends at an incline to the longitudinal direction of the tubular housing (2) and adjoins the stop (50) of the switching element (40).

16. Surgical obturator in accordance with claim 15, **characterized in that** the flanks (58, 59) of the teeth (34, 42) are so designed that a rotation of the switching element (40) in the second direction under the influence of the stop (46) of the blade carrier (20) abutting on the fourth section (51) is limited such that the blade carrier (20), in relation to its position with the two stops (46, 50) abutting on each other, is retracted only slightly as far as its cutting position.

17. Surgical obturator in accordance with claim 16, **characterized in that** the engagement of the teeth (34, 42) of the blade carrier (20) and the protective cap (27) is released upon displacement of the protective cap (27) into the rest position, so that the limitation of the rotatability of the switching element (40) is eliminated.

18. Surgical obturator in accordance with claim 17, **characterized in that** the cam track (45) has a fifth section (52) which adjoins the inclined fourth section (51) and along which the front cam (46) of the blade carrier (20) slides upon retraction of the blade carrier (20) from the cutting position to the protected position and thereby rotates the switching element (40) in the second direction into the initial position for the next work cycle.

19. Surgical obturator in accordance with any one of claims 11 to 18, **characterized in that** when the switching element (40) is located in the blocking position and the stop (46) of the blade carrier (20) is in abutment with the fourth section (51), the rear cam (47) of the blade carrier (20) is located opposite a sixth section (53) of the cam track (45), which prevents a displacement of the blade carrier (20) in the direction from the protected position to the cutting position.

## Revendications

1. Obturateur chirurgical (1) pour transpercer ou perforer une paroi corporelle, comprenant un boitier (2) de forme tubulaire, un support de lame (20), qui peut coulisser en direction longitudinale dans le boitier (2) de forme tubulaire, entre une position de coupe avancée et une position de protection retirée vers l'arrière, et sur lequel est maintenue une lame coupante (22) avec un tranchant de coupe (23) guidé dans un guide de tracé correspondant du boitier (2) de forme tubulaire, l'obturateur comprenant également un cabochon de protection (27) qui surmonte la lame coupante (22) et peut coulisser dans le boitier (2) de forme tubulaire, en direction longitudinale, entre une position de repos avancée, dans laquelle la lame coupante (22) est totalement recouverte dans sa position de protection, et une position de travail retirée en arrière, ce cabochon de protection présentant une fente (29) à travers laquelle passe le tranchant de coupe (23) de la lame coupante (22) en faisant saillie du cabochon de protection (27) dans la position de travail de celui-ci, et l'obturateur comprenant, en outre, un dispositif de retrait vers l'arrière, qui est agencé dans le boitier (2) de forme tubulaire et fait coulisser la lame coupante (22) de la position de coupe dans la position de protection, et peut être activé par le coulissement du cabochon de protection (27) de la position de travail à la position de repos,
**caractérisé en ce que** le dispositif de retrait vers l'arrière comporte un élément de commande (40) qui est monté dans le boitier (2) de forme tubulaire, librement rotatif autour de l'axe longitudinal du boitier (2) de forme tubulaire et de manière non coulissante dans la direction longitudinale, et qui fixe la lame coupante (22) dans la position de coupe et la libère en étant activé pour un coulissement dans la position de repos, cet élément de commande étant susceptible, dans le cas d'un coulissement du support de lame (20) et/ou du cabochon de protection (27) dans la direction longitudinale du boitier (2) de forme tubulaire, de tourner par l'intermédiaire d'un guidage à came (45, 46, 47 ; 34, 42) agissant entre l'élément de commande (40) d'une part et le support de lame (20) et/ou le cabochon de protection (27) d'autre part, et **en ce que** le cabochon de protection (27) et le support de lame (20) sont sollicités par un ressort (36) commun, qui les écarte l'un de l'autre, et qui fait coulisser le cabochon de protection (27) de la position de travail à la position de repos, et le support de lame (20) de la position de coupe à la position de protection.

2. Obturateur chirurgical selon la revendication 1, **caractérisé en ce que** le ressort (36) est un ressort hélicoïdal agencé à l'intérieur du support de lame (20) et s'appuyant, à une extrémité, contre une protubérance (38) du cabochon de protection (27), qui s'engage à travers la paroi du support de lame (20) dans l'espace intérieur (35) de celui-ci.

3. Obturateur chirurgical selon la revendication 1 ou la revendication 2, **caractérisé en ce que** le tranchant de coupe (23) de la lame coupante (22) présente une forme de ligne en hélice, de sorte que le support de lame (20), lors d'un coulissement dans la direction longitudinale par rapport au boitier (2) de forme tubulaire, et le cabochon de protection (27), lors d'un coulissement longitudinal par rapport au support de lame (20), sont tournés relativement l'un par rapport à l'autre autour de l'axe longitudinal du support de lame (20).

4. Obturateur chirurgical selon l'une des revendications précédentes, **caractérisé en ce que** le support de lame (20) présente un corps allongé (19) en forme de douille, qui est agencé concentriquement au boitier (2) de forme tubulaire.

5. Obturateur chirurgical selon l'une des revendications précédentes, **caractérisé en ce que** le support de lame (20) est relié à une tige de poussée (6), qui s'étend à travers le boitier (2) de forme tubulaire jusqu'à un dispositif de pression (5) à l'extrémité du boitier (2) de forme tubulaire, qui est opposée à celle où se trouve la lame coupante (22).

6. Obturateur chirurgical selon l'une des revendications précédentes, **caractérisé en ce que** l'élément de commande (40) est une douille entourant de manière concentrique le support de lame (20).

7. Obturateur chirurgical selon la revendication 6, **caractérisé en ce que** le guidage à came agissant entre l'élément de commande (40) et le support de lame (20), est formé par des chemins de came (45) et des cames (46, 47) pouvant y être appliquées, qui sont agencés sur le côté extérieur du support de lame (20) respectivement la paroi intérieure de l'élément de commande (40) en forme de douille.

8. Obturateur chirurgical selon la revendication 7, **caractérisé en ce que** le long de la périphérie du support de lame (20) et de l'élément de commande (40) en forme de douille, sont agencés plusieurs chemins de came (45) et cames (46, 47) identiques, de façon telle que les cames (46, 47), en cas de cycles de travail successifs s'appuient contre un chemin de came (45) respectivement voisin.

9. Obturateur chirurgical selon l'une des revendications 7 ou 8, **caractérisé en ce qu'**à chaque chemin de came (45) sont associées deux cames (46, 47), qui, lors du coulissement du support de lame (20) par rapport à l'élément de commande (40), s'appuient successivement contre le chemin de came (45).

10. Obturateur chirurgical selon l'une des revendications précédentes, **caractérisé en ce que** le chemin de came (45) est formé par le bord d'une protubérance (44) en forme d'ilot.

11. Obturateur chirurgical selon l'une des revendications 7 à 10, **caractérisé en ce qu'**une came avant (46) dirigée vers la lame coupante (22), lors du coulissement du support de lame (20) de la position de protection en direction de la position de coupe, glisse le long d'un premier tronçon (48) du chemin de came (45), qui s'étend de manière inclinée par rapport à la direction de coulissement du support de lame (20), en faisant ainsi tourner l'élément de commande (40) dans une première direction à partir d'une position de base, et **en ce qu'**une came arrière (47) éloignée de la lame coupante (22), vient se mettre en appui contre un deuxième tronçon (49) qui s'étend de manière inclinée, dans la direction opposée, par rapport à la direction de coulissement du support de lame (20), dès que la came avant (46) a été poussée au-delà du premier tronçon (48) du chemin de came (45), de sorte que l'élément de commande (40) est tourné en direction opposée dans une position de blocage, dans laquelle, lors du retrait suivant, vers l'arrière, du support de lame (20) à partir d'une position extrême, qui se situe devant la position de coupe, une butée (46) agencée sur ce support de lame, vient en contact avec une butée (50) sur l'élément de commande (40) en empêchant ainsi un retrait supplémentaire vers l'arrière du support de lame (20).

12. Obturateur chirurgical selon la revendication 11, **caractérisé en ce que** la butée sur le support de lame (20) est formée par la came avant (46).

13. Obturateur chirurgical selon la revendication 11 ou la revendication 12, **caractérisé en ce que** la butée sur l'élément de commande (40) est formée par un troisième tronçon (50) du chemin de came (45), qui s'étend transversalement à la direction longitudinale du boitier (2) de forme tubulaire.

14. Obturateur chirurgical selon l'une des revendications 6 à 13, **caractérisé en ce que** le chemin de came et les cames entre l'élément de commande (40) et le cabochon de protection (27) sont formés par les flancs latéraux (56, 57, 58, 59) de dents (34, 42), qui sont agencées sur les faces frontales dirigées l'une vers l'autre du cabochon de protection (27) et de l'élément de commande (40), et qui sont en prise lorsque le cabochon de protection (27) est retiré vers l'arrière dans la position de travail.

15. Obturateur chirurgical selon l'une des revendications 11 à 13 et selon la revendication 14, **caractérisé en ce que** les flancs (56, 57) des dents (34, 42) sont agencées et configurées de manière telle, que les dents (34) du cabochon de protection (27), lors du retrait vers l'arrière du cabochon de protection (27) de la position de repos vers la position de travail, font tourner l'élément de commande (40) dans la première direction à partir de sa position de blocage, jusqu'à ce que la butée (46) du support de lame (20) parvienne sur un quatrième tronçon (51) du chemin de came (45) de l'élément de commande (40), qui s'étend de manière inclinée par rapport à la direction longitudinale du boitier (2) de forme tubulaire et se raccorde à la butée (50) de l'élément de commande (40).

16. Obturateur chirurgical selon la revendication 15, **caractérisé en ce que** les flancs (58, 59) des dents (34, 42) sont configurées de façon à ce qu'une rotation de l'élément de commande (40) dans la deuxième direction, sous l'influence de la butée (46) du support de lame (20) appliquée contre le quatrième tronçon (51), soit limitée de manière telle, que le support de lame (20) ne soit que très peu repoussé vers l'arrière, par rapport à sa position correspondant à l'appui réciproque des deux butées (46, 50) l'une contre l'autre, jusqu'à sa position de coupe.

17. Obturateur chirurgical selon la revendication 16, **caractérisé en ce que** la prise réciproque des dents (34, 42) du support de lame (20) et du cabochon de protection (27) est interrompue lors du coulissement du cabochon de protection (27) dans la position de repos, de sorte que la limitation de la possibilité de rotation de l'élément de commande (40) est supprimée.

18. Obturateur chirurgical selon la revendication 17, **caractérisé en ce que** le chemin de came (45) présente un cinquième tronçon (52), qui se raccorde au quatrième tronçon (51) incliné, et le long duquel glisse la came avant (46) du support de lame (20), lors du retrait vers l'arrière du support de lame (20) de la position de coupe vers la position de protection, en faisant tourner à cette occasion l'élément de commande (40), dans la deuxième direction, jusqu'à la position de base pour le cycle de travail suivant.

19. Obturateur chirurgical selon l'une des revendications 11 à 18, **caractérisé en ce que** la came arrière (47) du support de lame (20), pour un positionnement de l'élément de commande (40) dans la position de blocage et pour un appui de la butée (46) du support de lame (20) contre le quatrième tronçon (51), est en regard d'un sixième tronçon (53) du chemin de came (45), qui empêche un coulissement du support de lame (20) dans la direction allant de la position de protection vers la position de coupe.
